# EUROPEAN PATENT APPLICATION

(11) **EP 1 459 736 A1**
(43) Date of publication of application: **22.09.2004**
(21) Application number: 03251574.4
(22) Date of filing: 14.03.2003
(51) Int. Cl.: A61K 7/48

(54) **Skin care composition that increase and repair skin barrier function**

(71) Applicant: The Procter & Gamble Company, Cincinnati, Ohio 45202 (US)
(72) Inventor: Evans, Erica Louise, Marlborough, Wiltshire SN8 2PA (GB); Matts, Paul Jonathan, Addelstone, Surrey KT15 2HT (GB)
(74) Representative: Wilding, Richard Alan

(57) **Abstract**

Skin care compositions comprising greater than 7% glycerine, a vitamin B₃ compound and a natural moisturising factor are provided. The skin care compositions of the present invention provide improved acute skin hydration in combination with increased skin barrier function repair that result in improved chronic skin hydration.

## Description

### Technical Field

The present invention relates to skin care compositions. More specifically, skin care compositions are provided comprising glycerine, a vitamin B₃ compound and a natural moisturising factor. The skin care compositions herein provide improved acute skin hydration in combination with increased skin barrier function repair that result in improved chronic skin hydration.

### Background

A wide variety of cosmetic compositions have been used to treat dry skin. These compositions typically contain lipophilic moisturizing agents that inhibit water loss via occlusion. These compositions can additionally comprise other skin benefit agents such as vitamins and humectants. Addition of these ingredients can increase the moisturisation of the skin, or create other effects on the skin such as desquamation.

However, moisturisation of the skin by occlusive inhibition of water loss may only lead to a temporary relief from dry skin. Repairing and increasing skin barrier function may result in the skin feeling moist for longer, and prevent excessive water-loss in those suffering from dry skin. Repairing and increasing the skin barrier function can also result in the skin repairing itself, leading to improved skin health. Compositions known in the art have attempted to do this using humectants and various skin actives, such as US 5,254,331. However, these compositions do not moisturise or repair and increase skin barrier function sufficiently to meet the requirements of those consumers suffering from very dry skin.

It is therefore desirable to provide skin care compositions that deliver high levels of moisturisation to the skin and have improved skin barrier function repair characteristics. It is further desirable to provide skin care compositions that provide moisturisation and skin barrier function repair with excellent application characteristics. Furthermore, it is desirable to provide skin care compositions that effectively repair skin barrier function via delivery of very high levels of moisturisers such as humectants.

### Summary

According to the present invention, skin care compositions comprising:
a) greater than 7% by weight of the composition of glycerine;
b) a vitamin B₃ compound;
c) a natural moisturising factor comprising aspartic acid, threonine, serine, glutamic acid, citrulline, proline, glycine, alanine, cysteine, valine, methionine, isoleucine, leucine, tyrosine, phenylalanine, tryptophan, lysine, ornithine, histidine, arginine, sodium-2-pyrrolidone-5-carboxylate, or mixtures thereof;
are provided. The skin care compositions of the present invention provide excellent moisturisation benefits with increased skin barrier function repair.

### Detailed Description

All weights, measurements and concentrations herein are measured at 25°C on the composition in its entirety, unless otherwise specified.

Unless otherwise indicated, all percentages of compositions referred to herein are weight percentages and all ratios are weight ratios.

Unless otherwise indicated, all molecular weights are weight average molecular weights.

Unless otherwise indicated, the content of all literature sources referred to within this text are incorporated herein in full by reference.

Except where specific examples of actual measured values are presented, numerical values referred to herein should be considered to be qualified by the word "about".

The term "safe and effective amount" as used herein, means an amount of an active ingredient high enough to modify the condition to be treated or to deliver the desired skin care benefit, but low enough to avoid serious side effects, at a reasonable benefit to risk ratio within the scope of sound medical judgment. What is a safe and effective amount of the active ingredient will vary with the specific active, the ability of the active to penetrate through the skin or hair, the age, health condition, and skin or hair condition of the user, and other like factors.

As used herein, "cosmetically acceptable" means that ingredients which the term describes are suitable for use in contact with the skin or hair of humans and lower animals without undue toxicity, incompatibility, instability, irritation, allergic response and the like.

The skin care compositions of the present invention comprise greater than 7% glycerine, a vitamin B₃ compound, and a natural moisturising factor (NMF). It has surprisingly been found that the combination of these ingredients, when applied to the skin, provide excellent moisturisation with significantly improved skin barrier function repair. The compositions herein are able to provide greater relief from dry skin over a longer period. This is of particular benefit for those consumers who suffer from chronic dry skin, whereby available moisturising compositions only temporarily moisturise, and do little to repair skin barrier function and prevent subsequent water loss. Without wishing to be bound by theory, the high level of glycerine which increases the moisturisation of the skin, is believed to allow the combination of a vitamin B₃ compound and an NMF to act in synergy to repair the skin barrier function, and prevent subsequent water loss from the skin.

The compositions of the present invention comprise greater than 7% glycerine. Preferably, the compositions of the present invention comprise from 10% up to 40%, more preferably from 12% to 30%, and more preferably still from 15% to 27% by weight of glycerine.

Without wishing to be bound by theory, it is believed that the high levels of glycerine herein are useful in combination with a vitamin B₃ compound and NMFs. It is believed that, central to the efficacy of this combination, is the concentration of glycerine, above 7%. Acute hydration of the stratum corneum provided by the levels of glycerine greater than 7% increases significantly the specific activity of proteases involved in the desquamatory process, promoting an increase in stratum corneum turnover rate. This is believed to result in a significant decrease in loose squame sheets and a corresponding increase in skin barrier function efficiency. A surprising consequence of this is increased percutaneous penetration of the vitamin B₃ compound and the NMFs. These compounds act to further increase skin barrier function repair, chronically improving the skin's ability to retain moisture. Subsequent application of high levels of glycerine further increases the hydration of the skin, and reinforces the repair of skin barrier function. The net result of the cooperation of the two functions is to dramatically increase skin hydration, and its ability to hold this moisture and, consequently, relief from very dry skin. It has also been found that greater than 7% glycerine also allows the transition of crystalline lipid structures within the stratum corneum lipids into liquid crystalline states. It is thought that structures of this type are more mobile and enhance penetration of actives such as the vitamin B₃ compound and NMF. This results in the increased penetration of these actives, and consequently improved repair of skin barrier function. Additionally, glycerine is a more powerful humectant per unit weight than any other, and in order to generate similar humectancy in the present compositions using other humectants would require at least twice as much humectant on weight for weight basis. This would result in the compositions being sticky when applied, which is not acceptable to consumers.

However, the compositions of the present invention may further comprise other humectants. Suitable humectants useful herein include polyhydric alcohols other than glycerine, guanidine; glycolic acid and glycolate salts (e.g. ammonium and quaternary alkyl ammonium); lactic acid and lactate salts (e.g. ammonium and quaternary alkyl ammonium); other alpha hydroxy acids such as malic acid, aloe vera in any of its variety of forms (e.g., aloe vera gel); hyaluronic acid, precursors and derivatives thereof (e.g., glucosamine and salt derivatives such as sodium hyaluronate); lactamide monoethanolamine; acetamide monoethanolamine; urea; and mixtures thereof. Preferably, the compositions of the present invention comprise less than 10%, more preferably less than 7% of these other humectants. It has surprisingly been found to be particularly disadvantageous to use 1,3-butylene glycol in the compositions of the present invention.

The skin care compositions of the present invention comprise a vitamin B₃ compound. Preferably the compositions of the present invention comprise from 2% to 5%, more preferably 2.5% to 4.5%, more preferably still from 3% to 4% of a vitamin B₃ compound. These levels of vitamin B₃ compound are preferred to deliver excellent benefits to the skin with regards to repairing skin barrier function. Vitamin B₃ compounds have been shown to have a breadth of cutaneous benefits, due to their known status as a precursor of nicotinamide cofactors such as NAD(H), NADP(H). The known benefits of niacinamide include upregulation of sphingolipid synthesis, including those ceramides critical to the formation of the lipid bilayer and so stratum corneum barrier integrity. The compositions of the present invention herein deliver superior skin moisturisation and skin barrier repair as a result of the synergistic action of the vitamin B₃ compound in combination with the NMF and high levels of glycerine.

As used herein, "vitamin B₃ compound" includes compounds having the formula: 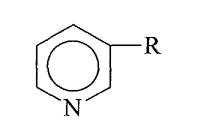 wherein R is - CONH₂ (i.e., niacinamide), - COOH (i.e., nicotinic acid) or - CH₂OH (i.e., nicotinyl alcohol); derivatives thereof; and salts of any of the foregoing. Exemplary derivatives of the foregoing vitamin B₃ compounds include 'non-vasodilating' esters of nicotinic acid, nicotinyl amino acids, nicotinyl alcohol esters of carboxylic acids, nicotinic acid N-oxide and niacinamide N-oxide. Preferably, the compositions herein comprise niacinamide. As used herein, "non-vasodilating" means that the ester does not commonly yield a visible flushing response after application to the skin in the subject compositions.

The skin care compositions of the present invention comprise a natural moisturising factor (NMF) comprising aspartic acid, threonine, serine, glutamic acid, citrulline, proline, glycine, alanine, cysteine, valine, methionine, isoleucine, leucine, tyrosine, phenylalanine, tryptophan, lysine, ornithine, histidine, arginine, sodium-2-pyrrolidone-5-carboxylate, or mixtures thereof. Preferably, the compositions of the present invention comprise at least 0.1%, more preferably from 0.1% to 5%, more preferably still from 0.2% to 4% by weight of the composition of NMF. Preferably, the skin care compositions herein comprise glycine, alanine, proline, serine, threonine, arginine, lysine, glutamic acid, sodium-2-pyrrolidone-5-carboxylate, or mixtures thereof. More preferably, the skin care compositions of the present invention comprise a mixture of glycine, alanine, proline, serine, threonine, arginine, lysine, glutamic acid, and sodium-2-pyrrolidone-5-carboxylate. Without wishing to be bound by theory, this mixture of natural moisturising factors appears to mimic the composition of NMFs found in healthy skin. The combination with high levels of glycerine and vitamin B₃ compound allows the NMFs herein to penetrate and repair skin barrier function more efficiently. Suitable, non-limiting examples of commercially available NMF compositions for use in the present invention include Prodew 400 ™, available from Ajinomoto, comprising a mixture of glycine, alanine, proline, serine, threonine, arginine, lysine, glutamic acid, and sodium-2-pyrrolidone-5-carboxylate.

Without wishing to be bound by theory, it is believed that NMFs are essential in the compositions of the present invention as they are an integral part of the epidermal barrier, residing in the bilayer and corneocyte structures of the stratum corneum. They appear to play a key role in barrier utility, facilitating water transfer to the keratinous structures in the stratum corneum (Denda, M. *et al.,* Stratum corneum sphingolipids and free amino acids in experimentally-induced scaly skin, Arch. Dermatol. Res., *284(6),* 363-367, 1992; Horii, 1. *et al.,* Stratum corneum hydration and amino acid content in xerotic skin, Brit. J. Dermatol., *121*, 587-592, 1989). Their use herein, in combination with vitamin B₃ compounds and high levels of glycerine is believed to result in a synergistic action on the skin, resulting in improved skin barrier function repair.

The skin care compositions of the present invention may further comprise a zwitterionic moisturising factor. Preferably, the compositions of the present invention comprise from 0.1 % to 5%, more preferably from 0.2% to 4%, more preferably still from 0.3% to 2% of a zwitterionic moisturising factor. Zwitterionic moisturising factors suitable for use herein include those with a molecular weight of from greater than 75 to less than 260, preferably from greater than 75 to less than 200 and more preferably from greater than 75 to less than 180. Zwitterionic moisturising factors are useful herein, as they provide immediate moisturisation of the skin, whilst also boosting skin barrier function and the osmo-competency of the skin.

Zwitterionic moisturising factors useful in the present invention comprise quaternary ammonium salts represented by the general formula (I) below: 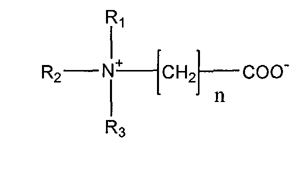 wherein R₁, R₂, and R₃ are independently selected from -H, -CH_{3,} -CH₂CH₃, and [-CH₂CH(OH)R_{4,} wherein R₄ is selected from -H, and CH₃]; and wherein n = an integer from 1 to 3, preferably 1. Preferably R₁, R_{2,} and R₃ are all -CH₃, and n is 1; or R₁, R_{2,} and R₃ are all -CH_{3,} and n is 2; or R₁ and R₂ are -CH_{3,} R₃ is -H, and n is 1; or R₁ is -CH_{3,} R₂ and R₃ are -H, and n is 1; or R₁ and R₂ are [-CH₂CH(OH)R_{4,} R₄ is -H or -CH₃], R₃ is -H, and n is 1.

Further zwitterionic moisturising factor derivatives suitable for use in the present invention are those comprising carnitine, trimethylamineoxide, tricine, and mixtures thereof. Preferred for use herein are zwitterionic moisturising factor or derivatives thereof comprising trimethylglycine, dimethyl glycine, sarcosine, trimethyl alanine, tricine, bicine, gamma-butyro betaine, trimethylamineoxide, carnitine, and mixtures thereof, more preferably comprising trimethylglycine, tricine and dimethyl glycine; and mixtures thereof, more preferably still comprising trimethylglycine.

The compositions of the present invention comprise a safe and effective amount of a dermatologically acceptable carrier within which the essential materials and optional other materials are incorporated to enable the essential materials and optional components to be delivered to the skin at an appropriate concentration. The carrier may be solid, semi-solid or liquid. Preferred carriers are substantially liquid. The type of carrier utilized in the present invention depends on the type of product form desired for the composition. The topical compositions useful in the subject invention may be made into a wide variety of product forms such as are known in the art. These include, but are not limited to, lotions, creams, gels, sticks, sprays, ointments, pastes, mousses, aerosols and cosmetics (e.g., foundations, eye-makeup, pigmented or non-pigmented lip treatments, e.g., lipsticks, and the like).

Preferred carriers comprise an emulsion comprising a hydrophilic phase and a hydrophobic phase. As is well known to one skilled in the art, the hydrophilic phase will be dispersed in the hydrophobic phase, or vice versa, to form respectively hydrophilic or hydrophobic dispersed and continuous phases, depending on the composition ingredients. The emulsion may be or comprise (e.g., in a triple or other multi-phase emulsion) an oil-in-water emulsion or a water-in-oil emulsion such as a water-in-silicone emulsion. Emulsions of the present invention may comprise one or more of the following:

### a) Hydrophobic component

Emulsions according to the present invention may comprise a hydrophobic phase comprising a lipid, oil, oily or other hydrophobic component. The compositions of the present invention preferably comprise from about 1% to about 50%, preferably from about 1% to about 30%, and more preferably from about 1% to about 15% by weight of the composition of a hydrophobic component. Nonlimiting examples of suitable hydrophobic components include those selected from the group consisting of:
(1) Mineral oil, which is also known as petrolatum liquid, is a mixture of liquid hydrocarbons obtained from petroleum.
(2) Petrolatum, which is also known as petroleum jelly, is a colloidal system of nonstraight-chain solid hydrocarbons and high-boiling liquid hydrocarbons,
(3) Straight and branched chain hydrocarbons having from about 7 to about 40 carbon atoms. Nonlimiting examples of these hydrocarbon materials include dodecane, isododecane, squalane, cholesterol, hydrogenated polyisobutylene, docosane (i.e. a C₂₂ hydrocarbon), hexadecane, isohexadecane (a commercially available hydrocarbon sold as Permethyl ™ 101A by Presperse, South Plainfield, NJ).
(4) C1-C30 alcohol esters of C1-C30 carboxylic acids and of C2-C30 dicarboxylic acids, including straight and branched chain materials as well as aromatic derivatives (as used herein in reference to the hydrophobic component, mono- and poly- carboxylic acids include straight chain, branched chain and aryl carboxylic acids). Nonlimiting examples include, isononylisononoate, diisopropyl adipate, isopropyl myristate, isopropyl palmitate, methyl palmitate, myristyl propionate, 2-ethylhexyl palmitate, isodecyl neopentanoate, di-2-ethylhexyl maleate, cetyl palmitate, myristyl myristate, stearyl stearate, isopropyl isostearate, methyl stearate, cetyl stearate, diisopropyl adipate, cetyl octanoate,.
(5) mono-, di- and tri- glycerides of C1-C30 carboxylic acids, e.g., caprilic/capric triglyceride, PEG-6 caprylic/capric triglyceride, PEG-8 caprylic/capric triglyceride.
(6) alkylene glycol esters of C1-C30 carboxylic acids, e.g., ethylene glycol mono- and di- esters, and propylene glycol mono- and di- esters of C1-C30 carboxylic acids e.g., ethylene glycol distearate.
(7) propoxylated and ethoxylated derivatives of the foregoing materials.
(8) Organopolysiloxane oils. The organopolysiloxane oil may be volatile, non-volatile, or a mixture of volatile and non-volatile silicones. The term "nonvolatile" as used in this context refers to those silicones that are liquid under ambient conditions and have a flash point (under one atmospheric of pressure) of or greater than about 100°C. The term "volatile" as used in this context refers to all other silicone oils. Examples of suitable organopolysiloxane oils include polyalkylsiloxanes, cyclic polyalkylsiloxanes, and polyalkylarylsiloxanes.
   Polyalkylsiloxanes useful in the composition herein can be represented by the general chemical formula R₃SiO[R₂SiO]ₓSiR₃ wherein R is an alkyl group having from one to about 30 carbon atoms (preferably R is methyl or ethyl, more preferably methyl; also mixed alkyl groups can be used in the same molecule), and x is an integer from 0 to about 10,000, chosen to achieve the desired molecular weight which can range to over about 10,000,000. Commercially available polyalkylsiloxanes include the polydimethylsiloxanes, which are also known as dimethicones, examples of which include the Dow Coming 200 series sold by Dow Coming Corporation.
   Cyclic polyalkylsiloxanes suitable for use in the composition include those represented by the chemical formula [SiR₂-O]ₙ wherein R is an alkyl group (preferably R is methyl or ethyl, more preferably methyl) and n is an integer from about 3 to about 8, more preferably n is an integer from about 3 to about 7, and most preferably n is an integer from about 5 to about 6. When R is methyl, these materials are typically referred to as cyclomethicones. Commercially available cyclomethicones include Dow Corning™ 245 and 246 fluids.
   Dimethiconols are also suitable for use in the composition. These compounds can be represented by the chemical formulas R₃SiO[R₂SiO]ₓSiR₂OH and HOR₂SiO[R₂SiO]ₓSiR₂OH wherein R is an alkyl group (preferably R is methyl or ethyl, more preferably methyl) and x is an integer from 0 to about 500, chosen to achieve the desired molecular weight. Commercially available dimethiconols are typically sold as mixtures with dimethicone or cyclomethicone (e.g. Dow Corning ™ 1503 fluid). Preferred for use herein are dimethicones, dimethiconols, and cyclomethicones.
(9) Cross-linked Siloxane Elastomers: Suitable organopolysiloxane gel compositions are dimethicone/vinyl dimethicone crosspolymers swollen in an appropriate solvent. Such dimethicone/vinyl dimethicone crosspolymers are supplied by a variety of suppliers including Dow Coming (DC 9040™ and DC 9041™), General Electric (SFE 839™), Shin Etsu (KSG-15™, KSG-16™, KSG-18™ [dimethicone /phenyl vinyl dimethicone crosspolymer]) and lauryl dimethicone/vinyl dimethicone crosspolymers supplied by Shin Etsu (e.g., KSG-31™, KSG-32™, KSG-41™, KSG-42™, KSG-43™, and KSG-44™). Alternatively, organopolysiloxane elastomer powders can be used, suitable examples include vinyl dimethicone/methicone silesquioxane crosspolymers like Shin-Etsu's KSP-100™, KSP-101™, KSP-102™, KSP-103™, KSP-104™, KSP-105™, hybrid silicone powders that contain a fluoroalkyl group like Shin-Etsu's KSP-200™, and hybrid silicone powders that contain a phenyl group such as Shin-Etsu's KSP-300™; and Dow Corning's DC 9506™.
(10) Vegetable oils and hydrogenated vegetable oils. Examples of vegetable oils and hydrogenated vegetable oils include those derived from safflower oil, castor oil, coconut oil, cottonseed oil, palm kernel oil, palm oil, peanut oil, soybean oil, rapeseed oil, linseed oil, rice bran oil, pine oil, sesame oil, sunflower seed oil, and mixtures thereof.

### b) Hydrophilic component

Emulsions of the present invention also comprise a hydrophilic component, e.g., water or other hydrophilic diluent. The hydrophilic phase can thus comprise water, or a combination of water and one or more water soluble or dispersible ingredients. Hydrophilic components comprising a substantial amount of water are preferred. The composition preferably comprises from about 10% to about 92.99% of the hydrophilic diluent.

### (c) Other components

Emulsions and other topical compositions of the present invention may comprise a variety of other ingredients such as disclosed herein. As will be understood by the skilled artisan, a given component will distribute primarily into either a hydrophilic phase or hydrophobic phase, depending on the hydrophilicity of the component in the composition.

Emulsions of the present invention preferably include one or more compounds selected from emulsifiers, surfactants, structuring agents, and thickeners;

### (1) Emulsifiers/Surfactants

The compositions of the present invention preferably comprise from about 0.05% to about 15% of a surfactant or mixture of surfactants. The exact surfactant or surfactant mixture chosen will depend upon the pH of the composition and the other components present.

Preferred surfactants are nonionic. Among the nonionic surfactants that are useful herein are those that can be broadly defined as condensation products of long chain alcohols, e.g. C₈₋₃₀ alcohols, with sugar or starch polymers, i.e., glucosides. These compounds can be represented by the formula (S)ₙ-O-R wherein S is a sugar moiety such as glucose, fructose, mannose, and galactose; n is an integer of from about 1 to about 1000, and R is a C₈₋₃₀ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples include a mixture of cetearyl glucosides and cetearyl alcohols such as those commercially available as Montanov 68™ from Seppic and Emulgade PL68/50™ available from Henkel.

Other useful nonionic surfactants include the condensation products of alkylene oxides with fatty acids (i.e. alkylene oxide esters of fatty acids). These materials have the general formula RCO(X)ₙOH wherein R is a C₁₀₋₃₀ alkyl group, X is -OCH₂CH₂- (i.e. derived from ethylene glycol or oxide) or -OCH₂CHCH₃- (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)ₙOOCR wherein R is a C₁₀₋₃₀ alkyl group, X is -OCH₂CH₂-(i.e. derived from ethylene glycol or oxide) or -OCH₂CHCH₃-(i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula R(X)ₙOR' wherein R is a C₁₀₋₃₀ alkyl group, X is -OCH₂CH₂- (i.e. derived from ethylene glycol or oxide) or -OCH₂CHCH₃- (i.e. derived from propylene glycol or oxide), and n is an integer from about 6 to about 100 and R' is H or a C10-30 alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula RCO(X)ₙOR' wherein R and R' are C₁₀₋₃₀ alkyl groups, X is -OCH₂CH₂ (i.e. derived from ethylene glycol or oxide) or -OCH₂CHCH₃- (derived from propylene glycol or oxide), and n is an integer from about 6 to about 100, examples of which include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Preferred among the nonionic surfactants are those selected from the group consisting of steareth-2, steareth-21, ceteareth-20, ceteareth-12, sucrose cocoate, steareth-100, PEG-100 stearate, and mixtures thereof.

Another emulsifier useful herein are fatty acid ester blends based on a mixture of sorbitan or sorbitol fatty acid ester and sucrose fatty acid ester, the fatty acid in each instance being preferably C₈-C₂₄, more preferably C₁₀-C₂₀. The preferred fatty acid ester emulsifier is a blend of sorbitan or sorbitol C₁₆-C₂₀ fatty acid ester with sucrose C₁₀-C₁₆ fatty acid ester, especially sorbitan stearate and sucrose cocoate. This is commercially available from ICI under the trade name Arlatone 2121.

Emulsions of the present invention may include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. A commercially available example is DC5225C™ from Dow Coming Corporation. Other examples include alkyl-modified dimethicone copolyols, i.e., compounds that contain C₂-C₃₀ pendant side chains. Still other useful dimethicone copolyols include materials having various cationic, anionic, amphoteric, and zwitterionic pendant moieties. Additionally, emulsifying silicone elastomer surfactants can be used such as KSG21™ from Shin-Etsu.

### (2) Structuring Agent

The compositions hereof, and especially the emulsions hereof, may contain a structuring agent. Structuring agents are particularly preferred in the oil-in-water emulsions of the present invention. Without being limited by theory, it is believed that the structuring agent assists in providing rheological characteristics to the composition which contribute to the stability of the composition. Preferred for use herein are for example, saturated and unsaturated fatty alcohols such as behenyl alcohol, cetyl alcohol and stearyl alcohol.

### (3) Thickening Agent (including thickeners and gelling agents)

The compositions of the present invention can also comprise a thickening agent, preferably from about 0.1% to about 5%, more preferably from about 0.1 % to about 3%, and most preferably from about 0.25% to about 2%, of a thickening agent. Nonlimiting classes of thickening agents include those selected from the group consisting of:
(i) Carboxylic Acid Polymers These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Eamples of carboxylic acid polymer thickeners useful herein are those selected from the group consisting of carbomers (available as the Carbopol 900™ series from B.F. Goodrich eg.Carbopol 954™), acrylates/C10-C30 alkyl acrylate crosspolymers (commercially available as Carbopol 1342™, Carbopol 1382™, Pemulen TR-1™, and Pemulen TR-2™, from B.F. Goodrich) and mixtures thereof.
(ii) Crosslinked Acrylate Copolymers These polymers comprise a blend of a water soluble anionic acrylic monomer, a water soluble non-ionic acrylate monomer and a bifunctional monomeric cross-linking agent. Suitable water soluble anionic acrylic based monomers include acrylic acid, methacrylic acid and mixtures thereof. Suitable water-soluble non-ionic acrylate-based monomers include acrylamide, methacrylamide, N-vinyl pyrolidone, water-soluble hydroxy-substituted acrylic or methacrylic esters or mixtures thereof. Suitable bifunctional monomeric cross-linking agents include di, tri and tetraethylenically unsaturated materials such as methylene bis acrylamide, divinylpyrroline and allyl (meth) acrylate or mixtures thereof. Commercial examples of co-polymer compositions suitable for use herein include the co-polymer compositions commercially available from BASF Corp. under the tradename Luvigel ™ EM and the copolymer compositions available from CIBA Speciality Chemicals, Macclesfield, UK, under the tradename Salcare SC91™.
(iii) Polyacrylamide Polymers Also useful herein are polyacrylamide polymers, especially anionic polyacrylamide polymers including substituted branched or unbranched polymers. These polymers can be formed from a variety of monomers including acrylamide and methacrylamide which are unsubstituted or substituted with one or two alkyl groups (preferably C₁ to C₅). Preferred are acrylate amide and methacrylate amide monomers in which the amide nitrogen is unsubstituted, or substituted with one or two C₁ to C₅ alkyl groups (preferably methyl, ethyl, or propyl), for example, acrylamide, methacrylamide, N-methacrylamide, N-methylmethacrylamide, N,N-dimethylmethacrylamide, N-isopropylacrylamide, N-isopropylmethacrylamide, and N,N-dimethylacrylamide. These polymers have a molecular weight greater than about 1,000,000 preferably greater than about 1,5000,000 and range up to about 30,000,000. Most preferred among these polyacrylamide polymers is the anionic polymer given the CTFA designation polyacrylamide and isoparaffin and laureth-7, available under the tradename Sepigel 305 from Seppic Corporation (Fairfield, NJ).

### Optional Components

The topical compositions of the present invention may comprise a wide variety of optional components, provided that such optional components are physically and chemically compatible with the essential components described herein, and do not unduly impair stability, efficacy or other use benefits associated with the compositions of the present invention. Optional components may be dispersed, dissolved or the like in the carrier of the present compositions.

A preferred skin active according to the present invention comprises panthenol or its derivatives. The panthenol and its derivatives include D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pyridoxin, pantoyl lactose and Vitamin B complex. Where present, the compositions of the present invention comprise at least 0.25 % panthenol or its derivatives, preferably at least 0.4%.

The compositions of the present invention may optionally include particulate materials. Particulate materials suitable herein include materials that are insoluble in both water and oil with a median particle size of from 1 µm to 50 µm. Preferably the compositions of the present invention comprise particulate materials having a refractive index of from about 1.3 to about 1.7, the particulate materials being dispersed in the composition and having a median particle size of from about 2 to about 30 µm.. Suitable particulate materials are organic or organosilicone or inorganic. Preferred particles are free-flowing, solid, materials. By "solid" is meant that the particles are not hollow. The void at the centre of hollow particles can have an adverse effect on refractive index and therefore the visual effects of the particles on either skin or the composition. Suitable organic particulate materials include those made of polymethylsilsesquioxane, polyamide, polythene, polyacrylonitrile, polyacrylic acid, polymethacrylic acid, polystyrene, polytetrafluoroethylene (PTFE) and poly(vinylidene chloride). Copolymers derived from monomers of the aforementioned materials can also be used. Inorganic materials include silica and boron nitride. Representative commercially available examples of useful particulate materials herein are Microthene FN510™, Tospearl 145™, Orgasol 2002™, Nylonpoly WL10™, Dry Flo™ or mixtures thereof. The compositions of the present invention can comprise from about 0.1% to about 5% by weight of particulate materials.

A further optional component may comprise sunscreening agents. Preferred among those sunscreens which are useful in the compositions of the invention are those selected from octylmethoxycinnamate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide and mixtures thereof.

Generally, the sunscreens can comprise from about 0.5% to about 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

Other optional materials herein include pigments that, where water-insoluble, contribute to and are included in the total level of oil phase ingredients. Pigments suitable for use in the compositions of the present invention can be organic and/or inorganic. Also included within the term pigment are materials having a low colour or lustre such as matte finishing agents, and also light scattering agents. Preferred examples are titanium and iron oxides and interference pearls eg. mica platelets coated with titanium and / or iron oxides.

### Examples

| **Oil in Water Moisturising Emulsions: Examples I to V** | | | | | |
|---|---|---|---|---|---|
| **INGREDIENTS** | **I** **% w/w** | **II** **% w/w** | **III** **% w/w** | **IV** **% w/w** | **V** **% w/w** |
| DEIONISED WATER | QS | QS | QS | QS | QS |
| DISODIUM EDTA | 0.1 | 0.1 | 0.1 | 0.1 | 0.1 |
| GLYCERINE | 7.1 | 10.0 | 25.0 | 7.1 | 15.0 |
| NIACINAMIDE | 2.0 | 3.5 | 5.0 | 2.0 | 3.5 |
| PANTHENOL | 0.5 | 0.5 | 1.0 | 0.5 | 0.5 |
| PRODEW 400¹ | 1.0 | 1.0 | 3.0 | 1.0 | 1.0 |
| SK INFLUX² | ----------- | ----------- | 1.0 | 1.0 | ----------- |
| EMULGADE ³ | 0.2 | 0.2 | 0.2 | 0.2 | 0.2 |
| ISOHEXADECANE | 6.0 | 6.0 | 6.0 | 6.0 | 3.0 |
| ETHYL PARABEN | 0.15 | 0.15 | 0.15 | 0.15 | 0.15 |
| COCONUT OIL FRACTIONATED | 0.2 | 0.2 | 3.0 | 0.2 | -------- |
| PROPYL PARABEN | 0.07 | 0.07 | 0.07 | 0.07 | 0.07 |
| STEARIC ACID | 0.1 | 0.1 | 0.1 | 0.1 | 1.0 |
| PEG-100 STEARATE | 0.1 | 0.1 | 0.1 | 0.1 | 0.1 |
| STEARYL ALCOHOL | 0.61 | 0.61 | 0.61 | 0.61 | 0.79 |
| CETYL ALCOHOL | 0.49 | 0.49 | 0.49 | 0.49 | 0.64 |
| BEHENYL ALCOHOL | 0.40 | 0.40 | 0.40 | 0.40 | 0.52 |
| ISOPROPYL ISOSTEARATE | 1.5 | 1.5 | 3.0 | 1.5 | 1.5 |
| DL- α TOCOPHEROL ACETATE | 0.25 | 0.25 | 0.5 | 0.25 | 0.25 |
| MINERAL OIL | ----------- | ----------- | 1.0 | ----------- | -------- |
| PETROLATUM | 1.5 | 1.5 | ----------- | 1.5 | 2.0 |
| LUVIGEL EM ⁴ | ----------- | 2.0 | 2.0 | ----------- | 0.5 |
| SEPIGEL 305⁵ | 1.5 | ----------- | ----------- | 1.5 | -------- |
| RHODASURF L7⁶ | ----------- | 0.0055 | 0.0055 | ----------- | 0.0055 |
| VOLPO 3 ⁷ | ----------- | 0.045 | 0.045 | ----------- | 0.045 |
| SODIUM HYDROXIDE | 0.011 | 0.011 | 0.011 | 0.011 | 0.011 |
| MICROTHENE FN510 ⁸ | 0.2 | 0.5 | 1.0 | 0.5 | 0.5 |
| DRY FLO PLUS ⁹ | ----------- | 0.5 | 2.0 | 0.5 | 1.0 |
| BENZYL ALCOHOL | 0.25 | 0.25 | 0.25 | 0.25 | 0.25 |
| DC 1503 ¹⁰ | 1.5 | 1.5 | 1.5 | 1.5 | 1.0 |
| PERFUME | 0.3 | 0.3 | 0.3 | 0.3 | 0.3 |
| SILVER PEARL¹¹ | ----------- | 0.3 | ----------- | ----------- | ----------- |
| GOLD PEARL¹¹ | ----------- | 0.2 | ----------- | ----------- | ----------- |
| RED PEARL ¹² | 2.0 | ----------- | ----------- | ----------- | ----------- |
| TiO2 GLW75CAP-MP¹³ | ----------- | ----------- | 0.175 | ----------- | ----------- |
| AVOBENZONE | ----------- | ----------- | ----------- | 1.50 | ----------- |
| OCTYL SALICYLATE | ----------- | ----------- | ----------- | 3.5 | ----------- |
| PHENYL BENZIMIDAZOLE SULPHONIC ACID | ----------- | ----------- | ----------- | 1.0 | ---------- |
| TEA | ----------- | ----------- | ----------- | 0.61 | ----------- |

| | | | | | |
|---|---|---|---|---|---|
| 1. Prodew 400: Supplied by Ajinomoto, Stubbenhuk 3, D-20459, Hamburg, Germany. | | | | | |
| 2. SK Influx: Supplied by Goldschmidt AG, Goldschmidtstrasse 100, D-45127 Essen, Germany. | | | | | |
| 3. Emulgade :Supplied by Cognis Deutchland GmbH, Paul-Thomas Strasse 56, D-40551 Dusseldorf, Germany. | | | | | |
| 4. Luvigel EM: Supplied by BASF Plc, PO Box 4-Earl Road, Cheadle Hulme, Cheshire SK8 6QG | | | | | |
| 5. Sepigel 305: Supplied by Seppic, 75 Quai D'Orsay, Paris | | | | | |
| 6. Rhodasurf : Supplied by Coldic, Staisty Close, Homewood Trading Estate, Chesterfield, Derbyshire S42 5UG | | | | | |
| 7. Volpo 3 : Supplied by Croda Oleochemicals, Cowick hall, Snaith Goole, East Yorkshire, Dn14 9AA | | | | | |
| 8. Microthene: Supplied by Equistar Chemicals, 1221 McKinney Street, Suite 700, Houston, TX 77252-2583 | | | | | |
| 9. Dry Flo : Supplied by National Starch Chemical Company, 10, Finderne Avenue, Bridgewater, NJ 08807, USA | | | | | |
| 10. DC 1503: Supplied by Dow Coming, Kings Court, 185 Kinds Rd, Reading, Berks, RGI 4EX | | | | | |
| 11. Silver and Gold 'Prestige' pearls: Supplied by Eckart Gmbh and Co., Kaiserstrasse 30, 90763 Fuerth, Germany. | | | | | |
| 12. Red 'Flamenco Summit' pearls: Supplied by Emrikweg, NL-2031 BT Haarlem, The Netherlands. | | | | | |
| 13. GLW75CAP-MP TiO2: Supplied by Kobo Products Inc., Montrose Avenue, South Plainfield, N.J. 07080, USA. | | | | | |

The compositions are made as follows:

A water phase is prepared by admixing all water soluble ingredients, except sodium hydroxide and Prodew 400, in water and heating to about 80°C. A second premix is prepared by admixing of the oil soluble ingredients except the silicone oil (DC1503) and heating also to around 80°C. The oil phase is added to the water phase and sheared to form an emulsion.

The emulsion is cooled to 60°C and the polymeric thickener (Luvigel EM) and associated anionic surfactants (oleth 3, laureth 7) are then added. Sodium hydroxide solution is then added to neutralise to pH 6-7.5, except for examples where sunscreens are included. At 45-50°C the benzyl alcohol, Prodew, DC1503, dyes and particles (including mica pearls, titanium dioxide and iron oxides) are added and the resulting product is sheared to ensure particle dispersion, deagglomeration and homogeneity. The composition can then be cooled to 40°C and perfume can be added. The product can then be prepared for packaging.

Example 5 can be packaged in a suitable can, pressurised with propane /butane / isobutene at 40psi and dispensed as a mousse.

| **Water in Silicone Moisturising Cream and Foundations: Examples VI to VIII** | | | |
|---|---|---|---|
| **INGREDIENTS** | **VI** **% w/w** | **VII** **% w/w** | **VIII** **% w/w** |
| DEIONISED WATER | QS | 10.0 | QS |
| TRISODIUM EDTA | ----------- | ----------- | 0.1 |
| DISODIUM EDTA | 0.1 | 0.1 | ----------- |
| GLYCERINE | 10.0 | 10.0 | 7.1 |
| NIACINAMIDE | 3.5 | 2.0 | 2.0 |
| PANTHENOL | 1.0 | ----------- | 0.5 |
| PRODEW 400 | 1.0 | 2.0 | 3.0 |
| SODIUM CHLORIDE | ----------- | ----------- | 2.0 |
| PHENOXYETHANOL | ----------- | ----------- | 0.25 |
| DC245 CYCLOMETHICONE¹ | ----------- | QS | 17.0 |
| DC5225C ² | ----------- | 4.5 | 13.0 |
| DC9040³ | 8.50 | 45.0 | ----------- |
| KSG21 ⁴ | 2.50 | 0.5 | ----------- |
| ABIL EM-97 ⁵ | 0.30 | ----------- | ----------- |
| ABIL EM-90 ⁶ | ----------- | 0.10 | ----------- |
| KF-6017 ⁷ | 0.25 | ----------- | ----------- |
| HYDROPHOBIC TALC | ----------- | ----------- | 4.1 |
| HYDROPHOBIC IRON OXIDES | ----------- | 2.25 | 1.0 |
| HYDROPHOBIC TiO₂ | ----------- | 8.25 | 8.25 |
| EA-209 ⁸ | 2.5 | ----------- | ----------- |
| HYDROPHOBIC MICA | ----------- | 2.5 | ----------- |
| ETHYL PARABENS | 0.1 | 0.2 | ----------- |
| PROPYL PARABENS | 0.1 | 0.1 | ----------- |
| METHYL PARABENS | | 0.1 | ----------- |
| BENZYL ALCOHOL | 0.25 | 0.25 | ----------- |
| PROPYLENE GLYCOL | 1.0 | ----------- | ----------- |
| ARACHIDYL BEHENATE | ----------- | ----------- | 0.3 |
| SYNTHETIC WAX | ----------- | ----------- | 0.1 |
| TIOVEIL⁹ | ----------- | 2.50 | ----------- |
| OCTYLMETHOXYCINNAMATE | ----------- | 1.50 | ----------- |

| | | | |
|---|---|---|---|
| 1, 2 &3: Supplied by Supplied by Dow Corning, Kings Court, 185 Kinds Rd, Reading, Berks, RGI 4EX | | | |
| 4 & 7: Supplied by Shin Etsu Silicones, Bolderweg 32, 1332 AV, Almere, The Netherlands | | | |
| 5 & 6: Supplied by Goldschmidt AG, Goldschmidtstrasse 100, D-45127 Essen, Germany. | | | |
| 8: Supplied by Kobo Products Inc., Montrose Avenue, South Plainfield, N.J. 07080, USA. | | | |
| 9: Supplied by Uniqema, Bebington, Wirral, Merseyside, CH62 4UF, UK. | | | |

The compositions are made as follows:

A water phase is prepared by admixing all water soluble ingredients in water and heating to about 50°C to dissolve. A second premix is prepared by dispersing any powders in cyclomethicone and silicone surfactants. High shear is required to deagglomerate any pigments (titanium or iron oxides) in the product. For Example VIII only, the oil phase is then heated to around 85°C and the waxes are then melted into the pre-mix. The pre-mix is then cooled to room temperature allowing crystallisation. A water in silicone emulsion is then formed by slowly adding the water pre-mix to the oil phase and mixing with high shear. The DC9040 and liquid sunscreens, if present, are then added and mixed to ensure homogeneity. The product can then be prepared for packaging.

## Claims

1. A skin care composition comprising:
a) greater than 7% by weight of the composition of glycerine;
b) a vitamin B₃ compound;
c) a natural moisturising factor comprising aspartic acid, threonine, serine, glutamic acid, citrulline, proline, glycine, alanine, cysteine, valine, methionine, isoleucine, leucine, tyrosine, phenylalanine, tryptophan, lysine, ornithine, histidine, arginine, sodium-2-pyrrolidone-5-carboxylate, or mixtures thereof.

2. A skin care composition according to claim 1 comprising from 2% to 5% by weight of the composition of vitamin B₃ compound.

3. A skin care composition according to claim 1 or claim 2 wherein the vitamin B₃ compound comprises niacinamide, nicotinic acid, tocopherol nicotinate and inositol hexanicotinate, or mixtures thereof, preferably niacinamide.

4. A skin care composition according to any one of the preceding claims comprising at least 0.1% by weight of the composition of natural moisturising factor.

5. A skin care composition according to any one of the preceding claims further comprising a zwitterionic moisturising factor comprising a quaternary ammonium salt represented by the general formula (I) below: 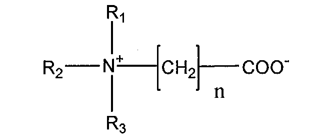 wherein R₁, R₂, and R₃ are independently selected from -H, -CH₃, - CH₂CH₃, and [-CH₂CH(OH)R₄, wherein R₄ is selected from -H, and CH₃]; and wherein n = an integer from 1 to 3, preferably 1, preferably trimethylglycine.

6. A skin care composition according to any one of the preceding claims additionally comprising a silicone phase.

7. A skin care composition according to any one of the preceding claims further comprising 0.1% to 5% thickening agent.

8. A skin care composition according to claim 7, wherein the thickening agent comprises carbomer, polyacrylamide, polyacrylates or mixtures thereof, preferably sodium acrylate copolymer, polyacrylamide, isoparaffin, laureth-7 co-polymer or mixtures thereof.

9. A skin care composition comprising;
a) from 10% to 15% by weight of the composition of glycerine;
b) from 2% to 5% by weight of the composition of niacinamide;
c) from 0.1% to 0.3% by weight of the composition of a natural moisturising factor comprising glycine, alanine, proline, serine, threonine, arginine, lysine, glutamic acid, sodium-2-pyrrolidone-5-carboxylate, or mixtures thereof.
